(19) Europäisches Patentamt / European Patent Office / Office européen des brevets

(11) **EP 2 678 735 B1**

(12) **FASCICULE DE BREVET EUROPEEN**

(45) Date de publication et mention de la délivrance du brevet:
**17.03.2021 Bulletin 2021/11**

(51) Int Cl.:
***G02C 13/00*** *(2006.01)*     ***A61B 3/11*** *(2006.01)*
***A61B 5/00*** *(2006.01)*

(21) Numéro de dépôt: **11815766.8**

(22) Date de dépôt: **30.12.2011**

(86) Numéro de dépôt international:
**PCT/FR2011/000688**

(87) Numéro de publication internationale:
**WO 2012/113995 (30.08.2012 Gazette 2012/35)**

(54) **PROCÉDÉ DE DÉTERMINATION D'AU MOINS UN PARAMÈTRE GÉOMÉTRICO-PHYSIONOMIQUE ASSOCIÉ AU MONTAGE D'UNE LENTILLE OPHTHALMIQUE DANS UNE MONTURE DE LUNETTES PORTÉE PAR UN PORTEUR**

VERFAHREN ZUR BESTIMMUNG VON MINDESTENS EINEM GEOMETRISCHEN/PHYSIOGNOMEN PARAMETER IM ZUSAMMENHANG MIT DER MONTAGE EINES BRILLENGLASES IN EINER VON EINEM BENUTZER GETRAGENEN BRILLENFASSUNG

METHOD FOR DETERMINING AT LEAST ONE GEOMETRIC/PHYSIOGNOMIC PARAMETER ASSOCIATED WITH THE MOUNTING OF AN OPHTHALMIC LENS IN A SPECTACLE FRAME WORN BY A USER

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **21.02.2011 FR 1100512**

(43) Date de publication de la demande:
**01.01.2014 Bulletin 2014/01**

(73) Titulaire: **Essilor International**
**94220 Charenton-le-Pont (FR)**

(72) Inventeurs:
• **HADDADI, Ahmed**
**F-94220 Charenton Le Pont (FR)**
• **DELZERS, Jean**
**F-94220 Charenton Le Pont (FR)**

(74) Mandataire: **Jacobacci Coralis Harle**
**32, rue de l'Arcade**
**75008 Paris (FR)**

(56) Documents cités:
**WO-A1-01/84222**     **WO-A1-2005/071468**
**WO-A1-2006/029875**     **WO-A1-2010/016379**
**WO-A2-2009/024681**

EP 2 678 735 B1

**Description**

DOMAINE TECHNIQUE AUQUEL SE RAPPORTE L'INVENTION

La présente invention concerne de manière générale un procédé de détermination d'au moins un paramètre géométrico-physionomique associé au montage d'une lentille ophtalmique dans une monture de lunettes portée par un porteur.

ARRIERE-PLAN TECHNOLOGIQUE

La confection d'une lentille correctrice de lunettes comporte, d'une part, la conception optique et la mise en forme des faces de réfraction de la lentille et, d'autre part, l'adaptation de la lentille à la monture choisie.

La présente invention traite de la mesure, sur le visage du porteur, de paramètres géométrico-physionomiques rendant compte de la configuration d'implantation des lunettes sur le visage du porteur. Ces paramètres sont susceptibles d'être exploités dans les deux étapes de confection d'une lentille correctrice, afin que la lentille exerce finalement la fonction optique corrective pour laquelle elle a été conçue et prescrite. Il s'agit notamment de la distance interpupillaire, de la hauteur des pupilles des yeux du porteur par rapport au bord inférieur de la monture, et/ou de l'angle pantoscopique que forme le plan général de la monture ou de la lentille par rapport à la verticale.

De manière connue, il est possible de déterminer ces paramètres à partir d'une ou plusieurs images capturées de la tête du porteur.

Il est également utile de déterminer le comportement optique du porteur, par exemple en déterminant l'orientation du regard et de la tête du porteur lorsque celui-ci suit des yeux un point en mouvement, par exemple pendant un mouvement de la tête de gauche à droite et/ou de droite à gauche. Pour ce faire, une acquisition video de plusieurs images pendant le mouvement de la tête est particulièrement adaptée.

La détermination des paramètres précités implique l'identification sur l'image capturée, de l'image d'au moins un élément de repère disposé sur le visage du porteur et présentant au moins une caractéristique géométrique prédéterminée.

Il est ensuite possible de déterminer le paramètre géométrico-physionomique recherché en comparant la caractéristique géométrique de l'image de l'élément de repère et sa caractéristique géométrique réelle correspondante.

Cependant, ce procédé ne peut être mis en oeuvre que si l'identification des éléments de repère sur l'image capturée est possible.

Or, un positionnement du porteur en contre-jour par rapport au dispositif de capture d'image peut conduire à la capture d'une image inexploitable, car trop sombre pour permettre l'identification de l'élément de repère.

Par ailleurs, lorsque la tête du porteur est éclairée au moins partiellement par la lumière du jour, l'éclairage de la tête du porteur peut varier, soit en fonction de la course du soleil dans le ciel, soit, lors d'une mesure dynamique, en fonction de la position de la tête du porteur par rapport aux sources lumineuses l'éclairant. Ainsi, les conditions d'éclairage de la tête du porteur peuvent être variables.

Les situations dans lesquelles les éléments de repère de la tête du porteur apparaissent trop sombres pour être identifiés précisément sur une image capturée sont donc multiples.

Une première solution à ce problème est de placer le dispositif de capture d'image dans un lieu dont l'éclairage est parfaitement contrôlé et constant. Cette solution a pour principal inconvénient de limiter considérablement les lieux propices à l'installation du dispositif permettant de mettre en oeuvre le procédé.

L'opticien peut alors être forcé de placer le dispositif de capture d'image dans une pièce aveugle. Il ne peut alors pas le placer en vitrine du magasin, alors que cela présente des avantages d'un point de vue commercial.

Une autre solution est d'utiliser un dispositif de capture d'image comportant des moyens de réglage automatique de la luminosité de l'image capturée. Cependant, ces moyens effectuent un réglage de la luminosité moyenne de l'ensemble de l'image, ce qui ne permet pas l'obtention d'une image exploitable dans le cas d'une capture d'image en contre-jour par exemple.

On connait du document WO01/84222 l'utilisation d'un procédé de réglage automatique des caractéristiques de l'image.

WO 2006/029875 A1 divulgue un accessoire avec des marques de repère comprenant deux zones contrastées qui sont utilisées pour déterminer la position de l'accessoire dans l'espace à partir d'une image de la tête du porteur équipé de l'accessoire. D'autres dispositifs de capture d'image connus comportent des moyens de réglage manuel de la luminosité. Il est alors possible de réaliser un ajustement du réglage de la luminosité avant chaque capture d'image. Cependant, cela présente l'inconvénient d'être long et fastidieux pour l'opticien. En outre, cela n'est pas réalisable dans le cadre d'une acquisition d'image continue en mode video.

OBJET DE L'INVENTION

Un but de la présente invention est donc de remédier aux inconvénients précités de l'état de la technique, en proposant un procédé de détermination d'au moins un paramètre géométrico-physionomique associé au montage d'une lentille ophtalmique dans une monture pouvant être mis en oeuvre quelles que soient les conditions de luminosité ambiante.

A cet effet, on propose selon l'invention un procédé tel que défini dans la revendication 1.

Ainsi, un réglage automatique des conditions optiques de capture d'image garantit de capturer une image exploitable pour la détermination du paramètre géométrico-physionomique recherché.

A cet effet, la luminosité de l'image est ajustée par la modification d'un paramètre de réglage des conditions optiques d'acquisition, en fonction de la luminosité de l'image d'un élément de calibrage de luminosité sur l'image capturée.

Dans un mode de réalisation préféré de l'invention, l'élément de calibrage est confondu avec un élément de repère dont les caractéristiques géométriques réelles connues et mesurées sur l'image sont exploitées pour déterminer le paramètre géométrico-physionomique recherché.

Le réglage de la luminosité est alors réalisé de manière à être optimisé directement sur l'image de cet élément de repère, ce qui garantit que l'image de cet élément de repère sera identifiable sur l'image capturée à l'étape f) avec le paramètre de réglage des conditions optiques d'acquisition modifié.

En variante, on peut prévoir que l'élément de calibrage est disposé à proximité de l'élément de repère utilisé à l'étape g) : le réglage de luminosité est alors réalisé pour une région de l'image très proche de l'élément de repère.

D'autres caractéristiques avantageuses et non limitatives de l'invention, sont énoncées aux revendications 2 à 14.

En outre, le procédé peut comporter une étape de ré-échantillonnage de l'image obtenue à l'étape b) destinée à réduire le nombre de pixels total de l'image.

DESCRIPTION DETAILLEE D'UN EXEMPLE DE REALISATION

La description qui va suivre, en regard des dessins annexés, donnée à titre d'exemple non limitatif, fera bien comprendre en quoi consiste l'invention et comment elle peut être réalisée.

Sur les dessins annexés :

- la figure 1 est un diagramme schématique des étapes d'un mode de réalisation possible de l'invention,
- la figure 2 est une vue schématique d'une image capturée par le dispositif de capture d'image,
- la figure 3 est une vue schématique en perspective d'un accessoire comportant des éléments de calibrage pour la mise en oeuvre du procédé selon l'invention,
- la figure 4A est une vue schématique de face d'un élément de calibrage de l'accessoire de la figure 3, visible au moins sur une vue de face de cet accessoire, et
- la figure 4B est une vue schématique de face d'un élément de calibrage de l'accessoire de la figure 3, visible au moins sur une vue de profil de cet accessoire,
- la figure 5 est une vue schématique des coefficients d'une matrice de renforcement utilisée dans le traitement de l'image capturée, comme expliqué ci-dessous, et
- la figure 6 est une vue schématique de la tête du porteur équipée de la monture de lunettes et de l'accessoire.

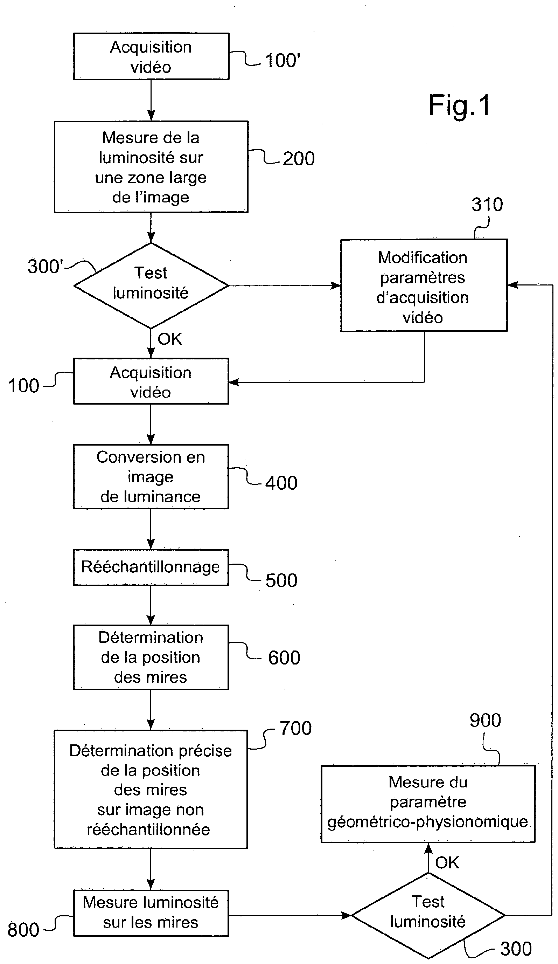

La figure 1 montre les étapes d'un mode de réalisation possible du procédé selon l'invention.

La mise en oeuvre de ce procédé a pour but la détermination d'au moins un paramètre géométrico-physionomique associé au montage d'une lentille ophtalmique dans une monture de lunettes portée par un porteur, tel que par exemple la distance interpupillaire, de la hauteur des pupilles des yeux du porteur par rapport au bord inférieur de la monture, et/ou de l'angle pantoscopique que forme le plan général de la monture ou de la lentille par rapport à la verticale.

Le procédé comporte les étapes suivantes, qui sont décrites ci-après plus en détail:

a) on place sur la tête du porteur un accessoire comportant au moins un élément de calibrage de luminosité comprenant au moins deux zones contrastées,

b) on capture une image de la tête du porteur sur laquelle est disposée ledit accessoire grâce à un dispositif de capture d'image,

c) on détermine la position de l'image de l'élément de calibrage de luminosité dudit accessoire sur ladite image capturée à l'étape b),

d) on détermine une valeur mesurée de la luminosité d'au moins une partie de l'image de cet élément de calibrage de luminosité ou d'au moins une partie de l'image située à proximité de cet élément de calibrage de luminosité,

e) on modifie un paramètre de réglage des conditions optiques d'acquisition d'image dudit dispositif de capture d'image en fonction de cette valeur mesurée de manière à améliorer le contraste de l'image des zones contrastées de l'élément de calibrage,

f) on capture une autre image avec le paramètre de réglage des conditions optiques d'acquisition modifié et

g) on détermine ledit paramètre géométrico-physionomique de montage recherché à partir de cette autre image.

<u>Etape a)</u>

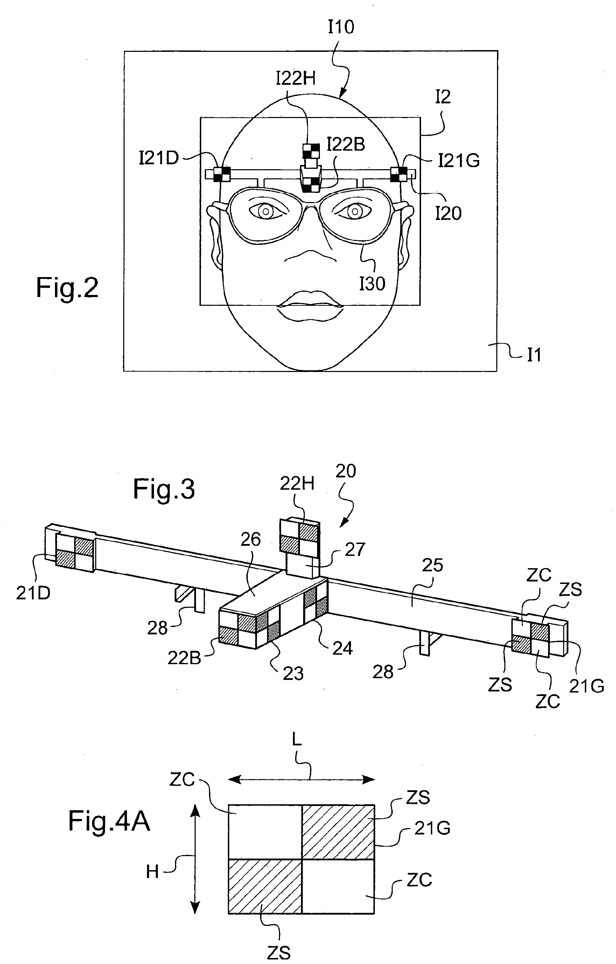

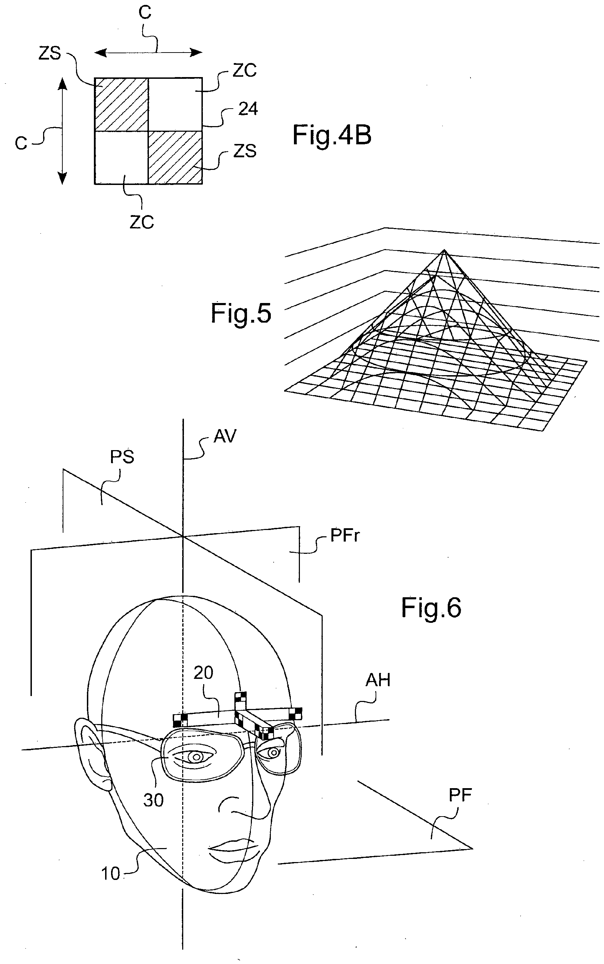

On place sur la tête 10 du porteur (figure 6) un accessoire 20, dont un exemple est représenté sur la figure 3.

L'accessoire 20 représenté sur la figure 3 est ici destiné à être monté sur la monture 30 de lunettes du porteur. Il comporte (figure 3) une barre principale 25 adaptée à être disposée au dessus de la monture de lunettes, dans un plan moyen des cercles de cette monture et de manière à s'étendre sensiblement parallèlement à une droite reliant les pupilles des yeux du porteur.

L'accessoire 20 dispose à cet effet de moyens de montage sur la monture 30 se présentant ici sous la forme de clips 28 s'étendant à partir de la barre principale 25.

Il est ici prévu deux clips 28, adaptés chacun à s'accrocher sur l'un des cercles de la monture 30.

Cet accessoire 20 comporte également une licorne 26 s'étendant perpendiculairement à la barre principale 25, dans un plan sensiblement perpendiculaire au plan moyen des cercles de la monture lorsque l'accessoire 20 est fixé sur cette monture 30, et un élément en saillie 27 s'élevant perpendiculairement à la barre principale 25 et à la licorne 26, dans le plan moyen de la monture 30 lorsque l'accessoire 20 est fixé sur cette monture.

Comme représenté sur la figure 6, on définit pour la tête du porteur un plan de Francfort PF passant par les points orbitaires inférieurs OR et le porion PO du porteur, le porion étant le point du crâne le plus élevé du conduit auditif, qui correspond au tragion de l'oreille. Ce plan de Francfort est sensiblement horizontal lorsque le porteur est en posture naturelle.

C'est le cas par exemple lorsque le porteur est dans une configuration assise ou debout dans laquelle sa tête 10 est droite et qu'il regarde droit devant lui, au loin, c'est-à-dire de préférence à l'horizon. L'axe de regard du porteur est alors horizontal.

On dit également que le porteur prend une position orthostatique, ou une position dans laquelle il réalise un minimum d'efforts.

On définit un plan sagittal PS comme étant le plan perpendiculaire au plan de Francfort passant par la médiatrice des deux yeux. La médiatrice des yeux est l'axe passant au milieu du segment défini par les centres de rotation CROG, CROD des deux yeux et parallèle au plan de Francfort PF.

On définit aussi un plan frontal PFr de la tête comme étant un plan perpendiculaire au plan de Francfort et parallèle à la droite reliant les deux yeux et passant par le sommet de la tête.

Dans ces conditions, un axe vertical AV de la tête 10 est défini comme l'intersection des plans frontal et sagittal et un axe horizontal AH de la tête est défini comme l'intersection des plans de Francfort et frontal. Cet axe horizontal AH est donc un axe perpendiculaire au plan sagittal PS de la tête du porteur.

Une image de face correspond alors à une image pour laquelle le plan de capture d'image du dispositif de capture d'image fait un angle compris entre +20 et -20 degrés avec le plan frontal PFr de la tête du porteur autour de l'axe vertical AV de la tête du porteur.

Une image de profil correspond alors à une image pour laquelle le plan de capture d'image du dispositif de capture d'image fait un angle compris entre +10 et -10 degrés avec le plan sagittal PS de la tête du porteur autour de l'axe vertical AV de la tête du porteur, ce qui correspond à un angle entre le plan frontal PFr et le plan de capture d'image compris entre 80 et 100 degrés.

L'accessoire 20 comporte au moins un élément de calibrage de luminosité 21D, 21G, 22H, 22B, 23, 24 comprenant au moins deux zones contrastées ZS, ZC.

Ici, l'accessoire 20 comporte huit éléments de calibrage 21D, 21G, 22H, 22B, 23, 24. Deux éléments de calibrage 21D, 21G sont disposés aux extrémités de la barre principale 25, et sont orientés de manière à être visibles au moins sur une image de face du porteur, lorsque l'accessoire 20 est fixé sur la monture du porteur.

Un élément de calibrage 22H est disposé sur l'élément en saillie 27 et un autre élément de calibrage 22B est disposé à l'extrémité de la licorne 26, de telle sorte que ces deux éléments de calibrage 22H, 22B sont visibles au moins sur une image de face du porteur. En outre, ces deux éléments de calibrage 22H, 22B sont disposés de telle sorte que, sur une vue de face de l'accessoire, ils sont situés l'un en dessous de l'autre.

Les éléments de calibrage 21D, 21G, 22H, 22B visibles au moins sur les images capturées de face sont appelés dans la suite éléments de calibrage de face.

Enfin les côtés latéraux de la licorne portent également chacun deux éléments de calibrage 23, 24 qui sont visibles au moins sur les images de profil du porteur et sont appelés dans la suite éléments de calibrage de profil.

En pratique, les éléments de calibrage 21G, 21D, 22H, 22B, 23, 24 sont visibles sur une large plage d'inclinaisons relatives entre la tête du porteur et le dispositif de capture d'image.

Chaque élément de calibrage comporte ici quatre zones contrastées ZS, ZC. Ces zones contrastées sont disposées en alternance, chaque zone formant un angle droit de sommet commun avec les angles droits formés par les autres zones. Ledit sommet commun des zones claires et sombres forme le centre de l'élément de calibrage.

Ces éléments de calibrage sont aussi appelés « mires », comme cela est le cas par exemple sur la figure 1.

En pratique, comme représenté plus en détail sur la figure 4A pour l'élément de calibrage 21G, chaque élément de calibrage de face 21D, 21G, 22H, 22B se présente ici sous la forme d'un rectangle de longueur L comprise entre 8 et 11 millimètres et de hauteur H comprise entre 5 et 8 millimètres.

En variante, lesdits éléments de calibrage peuvent présenter toute autre forme, notamment une forme carré ou circulaire.

Ce rectangle est divisé en quatre rectangles plus petits de dimensions égales. Les plus petits rectangles présentent deux à deux en diagonal des luminances ou des couleurs identiques, et présentent deux à deux avec leur voisin des luminances ou des couleurs différentes.

Comme représenté plus en détail sur la figure 6B pour l'élément de repère de profil 23, les éléments de repère de profil 23, 24 présentent de préférence une forme carrée avec un côté de longueur C égal à 6 millimètres. Ce carré est divisé en quatre carrés plus petits de dimensions égales. Les plus petits carrés présentent deux à deux en diagonal des luminances ou des couleurs identiques, et présentent deux à deux avec leur voisin des luminances ou des couleurs différentes.

Sur l'exemple représenté sur les figures 3, 4A et 4B, les éléments de calibrage comportent des zones contrastées en luminance : il s'agit ici de zones sombres et de zones claires de même couleur. Plus précisément, les zones contrastées ZS, ZC sont ici des zones de gris clair et de gris foncé non saturés. Dans ce cas, les éléments de calibrage permettent un calibrage de luminance et on entend par luminosité une valeur de luminance.

La répartition de luminance est en outre inversée entre les éléments de calibrage de face et de profil (figure 4A et 4B).

Dans un codage de couleur de type Rouge Vert Bleu, les zones de gris clair correspondent par exemple à trois composantes d'intensité égale à 225 et les zones de gris foncé correspondent à trois composantes d'intensité égale à 51.

De préférence, les zones contrastées ZS, ZC ne sont pas saturées, c'est-à-dire qu'elles ne sont pas noires et blanches.

En variante, lesdites zones contrastées de l'élément de calibrage comportent des zones de couleurs différentes. Ce sont alors des zones contrastées en chrominance. On peut par exemple envisager un élément de calibrage tout à fait similaire à celui décrit précédemment, dans lequel il est prévu une alternance de zones rouges et de zones vertes.

Dans ce cas, l'élément de calibrage permet un calibrage de chrominance et on entend par luminosité une valeur de chrominance.

Ces zones peuvent alors avoir des luminances identiques ou être également contrastées en luminance.

La forme rectangulaire de l'élément de calibrage, avec une longueur plus grande selon l'axe de la barre principale 25 de l'accessoire 20 permet d'assurer une détection efficace de cet élément de calibrage même lorsque le porteur bouge la tête 10 (voir figure 5), notamment selon un mouvement latéral de gauche à droite ou de droite à gauche autour de l'axe vertical AV.

Par exemple, avec un élément de calibrage mesurant 10 millimètres de longueur et 7 millimètres de hauteur, si la tête du porteur tourne d'un angle de 45 degrés autour de son axe vertical AV par rapport à l'orientation de la tête lorsque la plan frontal de celle-ci est parallèle au plan de capture d'image du dispositif de capture d'image, l'image de élément de calibrage sur l'image de la tête du porteur capturée par le dispositif de capture d'image mesure alors 7 millimètres de longueur sur 7 millimètres de haut, ce qui assure une identification aisée de l'image de cet élément de calibrage.

Dans l'exemple décrit ici, l'accessoire 20 comporte des éléments de calibrage 21D, 21G, 22H, 22B qui sont visibles au moins sur les images capturées de face de la tête du porteur et des éléments de calibrage 23, 24 visibles au moins sur les images capturées de profil.

En pratique, il est avantageusement possible d'identifier aisément au moins un élément de calibrage de face de l'accessoire sur une large plage d'orientation de la tête 10 par rapport au dispositif de capture d'image, par exemple sur une plage d'inclinaison du plan frontal PFr de la tête par rapport au plan de capture d'image comprise entre - 45 et + 45 degrés autour de l'axe vertical AV de la tête du porteur.

Cette plage d'inclinaison est d'autant plus grande que la longueur des éléments de calibrage dans la direction de la barre principale 25 de l'accessoire. Il est donc possible d'ajuster les dimensions des éléments de calibrage en fonction de la plage d'inclinaisons relative du plan frontal PFr de la tête et du plan de capture d'image prévue pendant la capture d'une série d'image donnée.

Les éléments de calibrage de face et de profil présentent des répartitions de zones contrastées différentes de manière à être aisément différenciés les uns des autres.

Dans l'exemple décrit ici, le réglage de la luminosité est réalisé grâce aux éléments de calibrage de face uniquement.

En variante, on peut envisager d'effectuer ce réglage de luminosité par rapport aux éléments de calibrage de face et de profil.

L'accessoire 20 comporte également des éléments de repère de position présentant des caractéristiques de forme et de couleur rendant la détermination de leur position sur l'image aisée et dont une caractéristique géométrique réelle est prédéterminée et connue.

Cette caractéristique géométrique réelle est comparée à la caractéristique géométrique correspondante de l'image de cet élément de repère pour en déduire un facteur d'échelle, et/ ou un angle d'orientation de la tête autour de son axe vertical AV, et/ou un angle d'orientation de la tête du porteur autour de l'axe horizontal AH.

Ici, avantageusement, les éléments de repère de position sont confondus avec les éléments de calibrage 21D, 21G, 22H, 22B, 23, 24. L'alternance des zones claires ZC et sombres ZS crée en effet une mire dont la position peut facilement être détectée, comme expliqué plus en détail ci-après, et dont les caractéristiques géométriques sont connues et peuvent être exploitées pour déterminer le paramètre géométrico-physionomique recherché.

En variante, on peut envisager que l'accessoire comporte au moins un élément de repère de position distinct des éléments de calibrage de luminosité.

Par exemple, l'élément de repère de position peut être une mire verte disposée à proximité de l'un des éléments de calibrage. La position relative de l'élément de calibrage par rapport à cet élément de repère est alors prédéterminée.

Etape b)

On capture une image de la tête du porteur sur laquelle est disposée ledit accessoire grâce à un dispositif de capture d'image.

Ce dispositif de capture d'image peut être tout dispositif de capture d'image connu, notamment un dispositif de capture d'image numérique.

Si le dispositif de capture d'image est un dispositif optique non numérique, l'image capturée est convertie en image numérique avant tout autre étape de traitement de cette image.

Le dispositif de capture d'image est de préférence un dispositif de capture d'image vidéo capturant entre 1 et 30 images par seconde. Compte tenu des aspects liés aux traitements des images, notamment du temps de calcul nécessaire pour traiter les images, de préférence entre 5 et 20 images par seconde sont capturées et traitées.

Le dispositif de capture d'image utilisé capture par exemple 15 images par secondes qui sont traitées par le procédé décrit ici.

Toutes les images capturées ne sont pas utilisées pour déterminer le paramètre géométrico-physionomique recherché, mais certaines d'entre elles permettent d'affiner les réglages du dispositif de capture d'image, comme expliqué ci-après, de manière à améliorer la luminosité des images capturées ultérieurement des éléments de repère de l'accessoire 20, de manière à assurer leur identification précise.

En pratique, on capture donc une série d'images à intervalle de temps sensiblement régulier (bloc 100 de la figure 1). Les paramètres de capture d'image du dispositif de capture sont modifiés selon les étapes décrites ci-après au fur et à mesure de la capture des images et de leur traitement.

Les images capturées sont ici par exemple des images noir et blanc dans lesquelles chaque pixel présente une luminance donnée.

Une telle image I1 est par exemple représentée sur la figure 2. Sur cette figure 2, l'image I1 est une image de face de la tête 10 du porteur. Elle comporte l'image de la tête I10, l'image de la monture I30, et l'image de l'accessoire I20 sur laquelle on peut identifier les images des éléments de calibrage I21D, I21G, I22H, I22B. Les images I23, I24 des éléments de calibrage de profil ne sont pas identifiables sur cette image de face.

On capture de manière générale une image de la tête du porteur ayant une orientation telle par rapport au dispositif de capture d'image qu'au moins un des éléments de calibrage décrits précédemment est visible sur l'image. Cette condition est vérifiée sur une très large plage d'inclinaisons relatives entre le plan frontal PFr de la tête du porteur et le plan de capture d'image, comme expliqué précédemment.

Cette image peut en particulier être une image de face de la tête du porteur, ou une image correspondant à une inclinaison relative du plan frontal PFr de la tête par rapport au plan de capture d'image comprise entre -45 et +45 degrés autour de l'axe vertical AV de la tête du porteur.

En variante, il est possible de capturer une image de profil du porteur.

En variante, on peut également envisager de capturer une image en couleur, c'est-à-dire dans laquelle chaque pixel contient une information de luminance et de chrominance, et convertir cette image en couleur en une image en tons de gris, ce qui est représenté sur le bloc 400 de la figure 1.

Pour cela, l'image capturée en codage (R,G,B) comportant des composantes rouge R, verte G, et bleu B est transformée de manière connue en coordonnées (Y,U,V) comportant des composantes de luminance Y et chrominance U,V. La luminance de chaque pixel est obtenue selon la formule :

$$Y = 0,299*R + 0,587*G + 0,114*B.$$

Etape c)

On détermine sur cette image la position de l'image d'au moins un des éléments de calibrage de luminosité 21D, 21G, 22H, 22B, 23, 24 dudit accessoire 20 sur l'image capturée à l'étape b), comme représenté sur le bloc 600 de la figure 1.

Pour cela, en pratique on réalise tout d'abord de préférence une étape de ré-échantillonnage de l'image obtenue à l'étape b) destinée à réduire le nombre de pixels total de l'image (bloc 500 de la figure 1).

Les étapes ultérieures de traitement de l'image décrites ci-dessous sont alors plus rapides, car les temps de calcul sont réduits avec la réduction du nombre de pixels de l'image.

Le coefficient de ré-échantillonnage est par exemple compris entre 1,5 et 3. Il est par exemple égal à 2.

En variante, il est évidemment possible d'utiliser l'image capturée à l'étape b) non ré-échantillonnée.

Pour déterminer la position de l'image de chaque élément de calibrage sur l'image, on détermine ici directement la position des images de ces éléments de calibrage par traitement de l'image capturée.

On pourrait en variante, comme évoqué précédemment, déterminer la position des images des éléments de repère de position de l'accessoire et en déduire la position des éléments de calibrage.

On réalise, à l'étape c), une étape i) de convolution d'au moins une partie de l'image capturée à l'étape b) par une matrice de détection reproduisant la répartition de luminosité attendue de l'image de l'un des éléments de calibrage.

Cette répartition de luminosité peut être une répartition de luminance ou de chrominance selon le type d'élément de calibrage utilisé, comme expliqué précédemment.

Ici, étant donnée l'agencement des zones sombres ZS et claires ZC de chaque élément de calibrage 21D, 21G, 22H, 22B, à l'étape i) de convolution, ladite matrice de détection est de la forme :

$$\begin{vmatrix} m & -m \\ -m & m \end{vmatrix}$$

Chaque élément m de la matrice de détection est une sous-matrice comportant un nombre de lignes et un nombre de colonnes tels que la matrice de convolution présente des dimensions inférieures ou égales aux dimensions correspondantes attendues en pixels de l'image de l'un des éléments de repère, en tenant compte d'un angle maximal envisagé entre le plan frontal de la tête du porteur et le plan de capture d'image.

En effet, comme évoqué précédemment, compte tenu de la rotation de la tête du porteur autour de son axe vertical AH, l'image de l'élément de calibrage I21D, I21G, I22H, I22B, I23, I24 (figure 2) ne présente pas nécessairement une forme rectangulaire.

Par exemple, si l'élément de calibrage mesure en réalité 10 millimètres de long sur 6 millimètres de hauteur, son image s'étend sur une image capturée en vue de face sur 20 pixels en longueur et 12 pixels en hauteur.

Sur une image capturée lorsque le plan frontal PFr de la tête du porteur est par exemple tourné de 45 degrés par rapport au plan de capture d'image PCI autour de l'axe vertical AV, l'image de l'élément de calibrage s'étend sur 12 pixels en longueur et 12 pixels en hauteur.

Après ré-échantillonnage, l'image de l'élément de calibrage sur l'image capturée à 45 degrés s'étend alors sur 6 pixels en longueur et 6 pixels en hauteur.

Chaque zone sombre ZS et chaque zone claire ZC de l'élément de calibrage s'étend donc sur 3 pixels en longueur et 3 pixels en hauteur.

Chaque élément m de la matrice de détection comporte alors par exemple 3 colonnes et 3 lignes afin que la taille de la matrice de détection reste inférieure ou égale à la taille de l'image, quelle que soit l'orientation de la tête du porteur par rapport au plan de capture d'image.

Dans l'exemple décrit ici, la luminosité de chaque zone sombre ou claire est uniforme, les coefficients de chaque élément m de la matrice de détection sont alors de préférence tous égaux.

En outre, la somme des coefficients positifs de la matrice de détection est de préférence égale à 1 afin d'éviter des phénomènes de saturation lors de la convolution.

Dans l'exemple présenté ici, la matrice m se présente alors sous la forme :

$$m = 1/18 * \begin{vmatrix} 1 & 1 & 1 \\ 1 & 1 & 1 \\ 1 & 1 & 1 \end{vmatrix}$$

La convolution de l'image capturée à l'étape b) par la matrice de détection est par exemple réalisée pour la totalité de l'image. Ceci permet d'identifier l'image de chaque élément de calibrage, sans connaître a priori la position de celui-ci dans l'image, ce qui est notamment le cas lors de la convolution de la première image d'une série d'images.

En pratique, ici, les deux éléments de repère de profil 23, 24 ne sont pas identifiés lors du traitement d'image décrit car ils présentent une répartition de luminance différente de celle des éléments de calibrage de face 21D, 21G, 22H, 22B, comme représenté sur les figures 3, 4A et 4B. Une détection sélective des éléments de calibrage de face et de profil peut donc être réalisée.

En pratique ici les zones claires et sombres sont inversées sur les éléments de repère de profil. La matrice de détection utilisée pour déterminer la position des éléments de repère frontaux n'est donc pas adaptée pour déterminer les éléments de repère de profil.

On pourrait également envisager de réaliser avant cette étape de convolution, une étape d'estimation approximative de la position des éléments de calibrage. Pour cela, on identifie sur l'image capturée l'image des pupilles des yeux du porteur et on en déduit une région de l'image dans laquelle chaque élément de calibrage a une forte probabilité de se trouver, connaissant par étalonnage la position approximative des éléments de repère par rapport aux pupilles du porteur.

Il est également possible d'effectuer un suivi de la position des éléments de calibrage : à partir de la position sur une image donnée de la tête du porteur de l'élément de calibrage, sa position approximative peut être estimée dans l'image suivante, par exemple en fonction de l'intervalle de temps entre deux captures d'image et de la vitesse moyenne de déplacement de la tête d'un porteur.

En pratique la vitesse de déplacement de la tête est déterminée par comparaison des deux images précédant la capture d'image réalisée.

Sur l'image suivante, l'élément de calibrage est ainsi situé dans une région centrée sur une zone correspondant à la position de l'image de l'élément de calibrage sur l'image précédente et dont le rayon est égal au déplacement maximal estimé de l'élément de calibrage entre deux captures d'image.

Seule une partie de l'image suivante, située dans la région déterminée précédemment est alors convoluée par la matrice de détection. Le calcul est ainsi plus rapide.

Dans l'exemple décrit mettant en oeuvre un élément de calibrage de luminance, la région de l'image convoluée par la matrice de détection comporte des groupes de pixels appartenant aux éléments de calibrage et d'autres groupes de pixels qui n'appartiennent pas aux éléments de calibrage.

Il est alors possible d'effectuer une étape j) de renforcement du contraste de l'image convoluée à l'étape i), au cours de laquelle on convolue au moins une partie de l'image capturée par une matrice de renforcement dont les dimensions sont inférieures ou égales aux dimensions correspondantes en pixels de l'image d'un élément de calibrage et dont les coefficients augmentent des bords vers le centre de la matrice de renforcement.

La répartition des coefficients d'une telle matrice est représentée schématiquement sur la figure 5, qui montre l'amplitude des coefficients en fonction de leur positions dans la matrice.

La taille de cette matrice de renforcement dépend de la taille de l'image de l'élément de calibrage, et donc du coefficient de ré-échantillonnage.

La convolution de l'image obtenue à l'étape i) par cette matrice de renforcement permet d'augmenter la luminance des pixels situés dans les zones dont la luminance suit la répartition attendue pour l'image de chaque élément de calibrage.

La détermination de la position des éléments de calibrage est ainsi plus précise.

On effectue ensuite, sur l'image obtenue à l'étape j), ou sur l'image obtenue à l'étape i) si la convolution par la matrice de renforcement de contraste n'est pas réalisée, une étape k) de recherche des maxima isolés.

Plus précisément, à l'étape k), on détecte des groupes de pixels présentant un pic de luminosité isolé et présentant une taille inférieure à la taille de l'élément de calibrage de luminosité dans toutes les directions.

Pour cela, on repère sur l'image obtenue à l'étape i) ou j) les pixels dont la luminosité est supérieure à une valeur seuil de luminosité prédéterminée.

Il peut bien entendu s'agir d'une valeur seuil de luminance ou de chrominance selon le type d'élément de calibrage utilisé.

On utilise ensuite un algorithme de remplissage d'une matrice d'isolement centrée sur chaque pixel repéré. Cet algorithme remplit la matrice avec les valeurs de luminosité des pixels voisins dudit pixel repéré si cette luminosité est supérieure à ladite valeur seuil.

La taille de la matrice d'isolement est choisie en fonction de la taille de l'image recherchée de l'élément de calibrage.

Si la matrice d'isolement reste vide le long de ses bords, ceci signifie que le groupe de pixels situé autour du pixel repéré correspond à un maximum isolé.

On effectue par exemple une première sélection parmi tous les groupes de pixels identifiés à l'étape k) en ne retenant que les dix groupes dont la luminosité est la plus grande.

On réalise ensuite une étape l) de sélection d'au moins deux groupes de pixels présentant un pic de luminosité isolé détectés à l'étape k) et présentant la plus forte probabilité d'être associés à l'image de deux des éléments de calibrage de luminosité de l'accessoire, à savoir au moins deux groupes de pixels pour lesquels est réduit l'écart entre

- une distance mesurée entre ces deux groupes et une distance de référence, et/ou
- un angle mesuré entre la droite passant par ces deux groupes et une direction de référence et un angle de référence, et/ou
- la luminosité mesurée au voisinage de ces deux groupes et une luminosité de référence, et/ou
- la différence de luminosité entre deux points de positions relatives prédéterminées par rapport à chaque groupe de pixels et une différence de luminosité de référence.

Pour cela, on détermine tous les couples de groupes de pixels présents sur l'image selon une direction donnée, par exemple en considérant un premier groupe de pixels et chaque autre groupe de pixels situé à gauche ou à droite de ce premier groupe, ou au-dessus ou en-dessous de ce premier groupe.

On attribue une note globale à chaque couple de groupes de pixels de manière à quantifier leur ressemblance avec l'image d'un couple d'éléments de calibrage donné de l'accessoire.

A titre d'exemple, on s'intéresse par exemple aux deux éléments de calibrage 21D, 21G disposés à chaque extrémité de la barre principale 25 de l'accessoire 20.

Un traitement similaire peut évidemment être fait pour les éléments de calibrage 22H, 22B disposés l'un en dessous de l'autre sur l'élément en saillie et la licorne de l'accessoire 20, ou pour les deux éléments de calibrage disposés de chaque côté de la licorne.

La note globale attribuée à chaque couple est déterminée en fonction de la comparaison de caractéristiques géométriques et de luminosité des groupes de pixels avec les caractéristiques attendues de l'image du couple d'éléments de calibrage considéré.

Par exemple, on compare la distance mesurée sur l'image entre les deux groupes de ce couple et la distance attendue entre les images des deux éléments de calibrage du couple considéré, et on attribue une première note intermédiaire aux couples considérés qui est d'autant plus grande que l'écart entre ces deux distances est faible. La distance attendue entre les images des deux éléments de calibrage est déterminée en fonction d'un facteur d'échelle prenant notamment en compte le ré-échantillonnage de l'image.

On compare également l'angle mesuré sur l'image entre la droite passant par ces deux groupes et une direction représentant par exemple l'image d'une droite horizontale et l'angle attendu entre la droite reliant les images des deux éléments de calibrage et l'image d'un plan horizontal. Une deuxième note intermédiaire d'autant plus grande que l'écart entre ces deux angles est petit est alors attribuée au couple considéré.

On compare également la luminosité mesurée au voisinage de ces deux groupes et une luminosité de référence déterminée en fonction des captures d'image précédentes.

Lorsqu'une série d'image est capturée, on peut supposer que les valeurs de luminosité mesurées sur deux images successives seront proches. Ainsi, on attribue au couple considéré une troisième note intermédiaire d'autant plus grande que l'écart de luminosité précité est petit.

Enfin, on peut ici par exemple comparer, pour chaque groupe de pixels dudit couple, une différence de luminosité de référence et la différence de luminosité entre deux points de positions relatives choisies pour qu'ils soient situés dans deux cadrans voisins et/ou dans deux cadrans diagonalement opposés de l'image de l'élément de repère dans le cas où ce groupe de pixels serait l'image d'un élément de repère.

Ici les luminosités des pixels situés dans deux cadrans voisins présentent une différence prédéterminée et les luminosités des pixels situés dans deux cadrans diagonalement opposés de l'élément de calibrage sont similaires. Les quatrièmes notes intermédiaires attribuées au couple sont donc déterminées en accord.

On peut également exclure de la sélection réalisée à l'étape I) les couples de deux groupes de pixels pour lesquels l'écart entre une distance mesurée entre ces deux groupes et une distance de référence, et/ou un angle mesuré entre la droite passant par ces deux groupes et une direction de référence et un angle de référence, et/ou la luminosité mesurée au voisinage de ces deux groupes et une luminosité de référence et/ou la différence de luminosité entre deux points de positions relatives par rapport à chaque groupe de pixels prédéterminées et une différence de luminosité de référence est supérieur à une valeur seuil.

Ceci revient, dans le cas de l'angle d'inclinaison, à considérer que l'inclinaison de la tête du porteur est limitée à une plage d'angles déterminée et à éliminer les couples de groupes de pixels pour lesquels l'angle mesuré entre la droite passant par ces deux groupes et une direction représentant par exemple l'image d'une droite horizontale implique que la tête du porteur n'est pas dans une posture naturelle.

On élimine par exemple les couples qui impliqueraient que la tête 10 du porteur est inclinée d'un angle supérieur à 20 degrés autour de l'axe horizontal AH de la tête du porteur.

La note globale de chaque couple est par exemple obtenue en multipliant toutes les notes intermédiaires attribuées à ce couple.

En variante, ces notes intermédiaires peuvent être pondérées selon l'importance ou la fiabilité du critère considéré.

Lesdites notes intermédiaires peuvent également être attribuées selon d'autres méthodes, par exemple en attribuant une note identique à tous les couples dont les caractéristiques décrites ci-dessus se trouvent dans un intervalle de valeurs prédéterminées.

Les notes intermédiaires peuvent également être calculées de manière à avoir une augmentation exponentielle en fonction de la réduction de l'écart entre les caractéristiques mesurées du couple et les valeurs de référence correspondantes.

Le couple de groupes de pixels présentant la meilleure note globale est identifié au couple d'images des éléments de calibrage recherché.

Dans le cas où cette détermination des positions des images des éléments de calibrage est réalisée sur une image ré-échantillonnée, on peut envisager de répéter sur l'image non ré-échantillonnée l'étape de convolution par une matrice de détection reproduisant la répartition de luminosité de l'image attendue de l'élément de calibrage, dans une zone réduite de l'image non ré-échantillonnée centrée autour de la position de l'image non ré-échantillonnée correspondant à chaque position déterminée pour les éléments de calibrage sur l'image ré-échantillonnée (bloc 700 de la figure 1).

La taille de la matrice de détection utilisée dans cette étape est alors adaptée à l'image non ré-échantillonnée.

Etape d)

On détermine une valeur mesurée de la luminosité (bloc 800 de la figure 1), luminance ou chrominance selon le type d'élément de calibrage utilisé, d'au moins une partie de l'image de cet élément de calibrage de luminosité ou d'au moins une partie de l'image située à proximité de cet élément de calibrage de luminosité.

La partie de l'image de l'élément de calibrage de luminosité pour laquelle on détermine une valeur mesurée de la luminosité est appelée dans la suite la zone de mesure. Elle est par exemple située à cheval sur lesdites deux zones contrastées de l'élément de calibrage.

La valeur de luminosité mesurée est une valeur moyenne sur l'ensemble de la zone de mesure.

Ici cette mesure est effectuée dans une zone à cheval sur les quatre zones contrastées, de préférence centrée sur le centre de l'élément de calibrage, qui correspond au pixel de luminosité maximale.

La zone de mesure de la luminosité est ici de forme carrée et de taille inférieure aux dimensions de l'image de l'élément de calibrage. L'utilisation d'une zone de mesure présentant ces caractéristiques rend la moyenne déterminée peu sensible à une erreur de centrage de cette zone de mesure par rapport au centre de l'élément de calibrage.

En variante, on détermine une valeur mesurée de la luminosité de chaque partie de l'image de cet élément de calibrage de luminosité et on calcule la moyenne de ces deux valeurs.

Etape e)

On modifie un paramètre de réglage des conditions optiques d'acquisition d'image dudit dispositif de capture d'image en fonction de cette valeur mesurée de manière à améliorer le contraste de l'image des zones contrastées ZS, ZC de l'élément de calibrage 21D, 21G, 22H, 22B, 23, 24.

Plus précisément, à l'étape e), on réalise un test de luminosité (bloc 300 de la figure 1):

- on détermine l'écart entre la valeur mesurée de la luminosité déterminée à l'étape d) et une valeur cible de luminosité,
- on compare cet écart avec une valeur maximale prédéterminée de cet écart,
- en fonction de cette comparaison, on modifie le paramètre de réglage des conditions optiques d'acquisition du dispositif de capture d'image de manière à faire tendre ladite valeur mesurée vers ladite valeur cible de luminosité.

La valeur cible de luminosité est prédéterminée par une étape d'étalonnage préalable. Elle peut par exemple être déterminée à partir d'une première image capturée dans des conditions de luminosité optimale ou être déterminée à partir d'une valeur moyenne de valeur mesurée de la luminosité sur des images exploitables.

En pratique, si cet écart est supérieur à une valeur maximale prédéterminée de cet écart, la modification du paramètre de réglage des conditions optiques d'acquisition (bloc 310 de la figure 1) du dispositif de capture d'image à l'étape e) est par exemple égale à la différence entre la valeur cible de luminosité et ladite valeur mesurée de la luminosité divisée par ladite valeur maximale prédéterminée de l'écart entre cette valeur cible de luminosité et cette valeur mesurée.

Si cet écart est inférieur à une valeur maximale prédéterminée de cet écart, la modification du paramètre de réglage est nulle et on peut alors passer directement à l'étape g) (bloc 900 de la figure 1).

Ladite valeur maximale prédéterminée de l'écart entre la valeur cible et la valeur mesurée de luminosité dépend par exemple du nombre de pas de réglage du paramètre de réglage des conditions optiques d'acquisition du dispositif de capture d'image. Plus ce paramètre de réglage comporte un grand nombre de pas de réglage, plus la valeur maximale prédéterminée de l'écart sera grande, afin de permettre néanmoins un réglage rapide de ce paramètre.

Dans l'idéal, la modification du paramètre de réglage dépend de la valeur de luminosité mesurée, de la valeur cible et de la valeur courante du paramètre de réglage.

Le lien entre la luminosité et le paramètre de réglage n'est pas forcément linéaire sur les caméras. Par exemple, une petite modification du paramètre de réglage lorsque l'image est saturée ou sous-exposée peut rester sans effet visible.

Ainsi, il peut être avantageux de faire varier la valeur maximale prédéterminée de l'écart en fonction de la valeur courante du paramètre de réglage. Le paramètre de réglage des conditions optiques d'acquisition du dispositif de capture d'image est un paramètre d'ouverture et/ou de gain et/ou de temps d'exposition.

En pratique, le dispositif de capture d'image est par exemple une caméra vidéo au format PAL entrelacé. La taille initiale de l'image, avant ré-échantillonnage est par exemple de 720x576 pixels.

On ajuste alors le paramètre « bright» de la caméra.

Le paramètre « bright» n'est pas un paramètre physique de la caméra, mais une consigne. La caméra est par exemple utilisée en mode de réglage semi-automatique. En fonction du paramètre « bright» demandé, la caméra ajuste alors automatiquement les trois paramètres matériels suivants :

- iris ou ouverture ou diaphragme,
- gain,
- temps de pause ou shutter.

Etape f)

On capture une autre image avec le paramètre de réglage des conditions optiques d'acquisition modifié (bloc 100 de la figure 1).

Etape g)

On détermine ledit paramètre géométrico-physionomique de montage recherché à partir de cette autre image (bloc 900 de la figure 1).

Pour cela, on identifie sur l'image capturée à l'étape f) au moins une caractéristique géométrique de l'élément de repère de l'accessoire.

Ici, on identifie une caractéristique géométrique de l'élément de calibrage de luminosité.

Pour déterminer par exemple la distance interpupillaire, il suffit de mesurer la distance séparant les pupilles sur l'image capturée à l'étape f), puis de déterminer un facteur d'échelle.

Ce facteur d'échelle est par exemple déterminé en comparant la longueur de l'élément de calibrage sur l'image et sa longueur réelle.

Le facteur d'échelle est ensuite appliqué à la distance interpupillaire mesurée sur l'image pour déterminer la distance interpupillaire réelle du porteur.

De préférence, après l'étape e), on répète les étapes b) à e) tant que la valeur mesurée à l'étape d) de la luminosité est hors d'un intervalle de valeurs prédéterminées, et on réalise les étapes f) et g) lorsque cette valeur mesurée est située à l'intérieur dudit intervalle prédéterminé.

Ainsi, la luminosité de l'image utilisée pour la détermination du paramètre géométrico-physionomique est optimale.

En variante, on peut prévoir de réaliser, avant l'étape b), un premier réglage grossier et rapide des paramètres d'acquisition du dispositif de capture d'image, par les étapes suivantes, représentées sur les blocs 100', 200 et 300' de la figure 1 :

     pa) on capture une image préliminaire de la tête du porteur,

     pb) on détermine une valeur mesurée de la luminosité moyenne d'au moins une zone réduite de cette image préliminaire, cette zone réduite étant adaptée à couvrir au moins une partie de l'image de la tête du porteur,

     pc) on ajuste grossièrement le paramètre de réglage des conditions optiques d'acquisition du dispositif de capture d'image en fonction de cette valeur mesurée de manière à améliorer le contraste de l'image de la tête du porteur capturée,

     pd) on utilise le paramètre de réglage des conditions optiques d'acquisition obtenu à l'étape pc) pour ajuster les conditions optiques de la capture d'image de l'étape b).

La zone réduite de l'image capturée à l'étape pa) utilisée à l'étape pb) est par exemple une zone centrale I2 (figure 2) de l'image I1 dont les dimensions sont égales à la moitié des dimensions de l'image totale. Cette zone centrale comporte généralement les yeux du porteur lorsque l'image est capturée de face.

En variante, si l'image capturée est une image de profil du porteur on peut envisager que la zone réduite utilisée à l'étape pb) est une moitié gauche ou droite de l'image.

On détermine donc à l'étape pb) une luminance ou une chrominance moyenne de cette zone réduite de l'image.

Plus particulièrement, à l'étape pc) :

-    on détermine l'écart entre la valeur mesurée déterminée à l'étape pb) et une valeur cible de luminosité,
-    on compare cet écart avec une valeur maximale prédéterminée de cet écart, et

en fonction de cette comparaison, à l'étape pc), on ajuste le paramètre de réglage des conditions optiques d'acquisition du dispositif de capture d'image de manière à faire tendre ladite valeur mesurée vers ladite valeur cible de luminosité.

Comme à l'étape e) décrite précédemment, la valeur cible de luminosité est prédéterminée par une étape d'étalonnage préalable.

En pratique, comme à l'étape e) la modification du paramètre de réglage des conditions optiques d'acquisition du dispositif de capture d'image à l'étape pc) est par exemple égale à la différence entre la valeur cible de luminosité et ladite valeur mesurée de la luminosité divisée par ladite valeur maximale prédéterminée de l'écart entre cette valeur cible de luminosité et cette valeur mesurée.

Ladite valeur maximale prédéterminée de l'écart entre la valeur cible et la valeur mesurée de luminosité dépend par exemple du nombre de pas de réglage du paramètre de réglage des conditions optiques d'acquisition du dispositif de capture d'image. Plus ce paramètre de réglage comporte un grand nombre de pas de réglage, plus la valeur maximale prédéterminée de l'écart sera grande, afin de permettre néanmoins un réglage rapide de ce paramètre.

Dans l'idéal, la modification du paramètre de réglage dépend de la valeur de luminosité mesurée, de la valeur cible et de la valeur courante du paramètre de réglage.

On peut également prévoir que les étapes pa) à pd) sont répétées tant que la valeur de luminosité moyenne mesurée sur la zone réduite de l'image considérée n'est pas comprise dans un intervalle de valeurs de luminosité prédéterminées.

Ainsi le réglage plus précis de la luminosité des éléments de calibrage pourra être réalisé plus rapidement.

On peut également envisager de répéter cette étape si la luminosité change brusquement au cours de l'acquisition d'une série d'images.

La présente invention n'est nullement limitée aux modes de réalisation décrits et représentés mais l'homme du métier saura y apporter toute variante conforme à son esprit.

On a décrit un exemple dans lequel la luminosité de l'élément de calibrage est déterminée sur une zone de mesure à cheval sur les zones contrastées de cet élément de calibrage.

On pourrait aussi travailler séparément sur les zones claires et sombres de l'élément de calibrage, en ayant deux valeurs cibles de luminosité et deux valeurs maximales de l'écart entre la valeur mesurée et la valeur cible.

Comme mentionné précédemment, il est également possible d'envisager de travailler avec des mires de couleur. Dans ce cas, il serait possible de travailler sur différents canaux de couleur, et donc de faire sur chacun un traitement similaire à celui en luminance.

En pratique, il serait préférable de choisir des éléments de calibrage rouge et vert et non bleus car les capteurs de caméra CCD y sont peu sensibles.

On peut enfin envisager que la mesure de luminosité réalisée déclenche l'allumage d'un moyen d'éclairement additionnel par exemple, à diodes électroluminescentes, disposé à proximité du dispositif de capture d'image, afin de modifier les conditions d'éclairage pendant la capture d'image suivante.

**Revendications**

**1.** Procédé de détermination d'au moins un paramètre géométrico-physionomique associé au montage d'une lentille ophtalmique dans une monture (30) de lunettes portée par un porteur, comportant les étapes suivantes :

  a) on place sur la tête (10) du porteur un accessoire (20) comportant au moins un élément de calibrage (21D, 21G, 22H, 22B, 23, 24) de luminosité comprenant au moins deux zones contrastées (ZS, ZC),

  b) on capture une image (I1) de la tête (10) du porteur sur laquelle est disposée ledit accessoire (20) grâce à un dispositif de capture d'image,

  c) on détermine la position de l'image de l'élément de calibrage (I21D, I21G, I22H, I22B, I23, I24) de luminosité dudit accessoire 20 sur ladite image I1 capturée à l'étape b),

  d) on détermine une valeur mesurée de la luminosité d'au moins une partie de l'image de cet élément de calibrage (I21D, I21G, I22H, I22B, I23, I24) de luminosité, en déterminant une valeur mesurée de la luminosité de chaque zone contrastée de l'image de cet élément de calibrage de luminosité et en calculant la moyenne de ces deux valeurs,

  e) on modifie un paramètre de réglage des conditions optiques d'acquisition d'image dudit dispositif de capture d'image en fonction de cette valeur mesurée de manière à améliorer le contraste de l'image des zones contrastées (ZS, ZC) de l'élément de calibrage,

  f) on capture une autre image avec le paramètre de réglage des conditions optiques d'acquisition modifié et

  g) on détermine ledit paramètre géométrico-physionomique de montage recherché à partir de cette autre image.

2. Procédé selon la revendication 1, selon lequel après l'étape e), on répète les étapes b) à e) tant que la valeur mesurée à l'étape d) de la luminosité est hors d'un intervalle de valeurs prédéterminées, et on réalise les étapes f) et g) lorsque cette valeur mesurée est située à l'intérieur dudit intervalle prédéterminé

3. Procédé selon l'une des revendications 1 et 2, selon lequel, l'accessoire comportant en outre un élément de repère présentant au moins une caractéristique géométrique connue, à l'étape g), on identifie sur l'image capturée à l'étape f) au moins une caractéristique géométrique de cet élément de repère de l'accessoire.

4. Procédé selon la revendication 3, dans lequel ledit élément de repère (21 D, 21 G, 22H, 22B, 23, 24) est confondu avec ledit élément de calibrage (21 D, 21 G, 22H, 22B, 23, 24) de luminosité.

5. Procédé selon l'une des revendications 1 à 4, selon lequel, à l'étape d), la partie de l'image de l'élément de calibrage de luminosité pour laquelle on détermine une valeur mesurée de la luminosité est située à cheval sur lesdites deux zones contrastées (ZS, ZC).

6. Procédé selon l'une des revendications 1 à 5, selon lequel, à l'étape e),

  - on détermine l'écart entre la valeur mesurée de la luminosité déterminée à l'étape d) et une valeur cible de luminosité,
  - on compare cet écart avec une valeur maximale prédéterminée de cet écart,
  - en fonction de cette comparaison, on modifie le paramètre de réglage des conditions optiques d'acquisition du dispositif de capture d'image de manière à faire tendre ladite valeur mesurée vers ladite valeur cible de luminosité.

7. Procédé selon l'une des revendications 1 à 6, comportant les étapes suivantes qui sont préliminaires à l'étape b) :

  pa) on capture une image préliminaire de la tête du porteur,

  pb) on détermine une valeur mesurée de la luminosité moyenne d'au moins une zone réduite de cette image préliminaire, cette zone réduite étant adaptée à couvrir au moins une partie de l'image de la tête du porteur,

  pc) on ajuste grossièrement le paramètre de réglage des conditions optiques d'acquisition du dispositif de capture d'image en fonction de cette valeur mesurée de manière à améliorer le contraste de l'image de la tête du porteur capturée,

  pd) on utilise le paramètre de réglage des conditions optiques d'acquisition obtenu à l'étape pc) pour ajuster les conditions optiques de la capture d'image de l'étape b).

8. Procédé selon la revendication 7, selon lequel, à l'étape pc),

  - on détermine l'écart entre la valeur mesurée de la luminosité déterminée à l'étape pb) et une valeur cible de luminosité,
  - on compare cet écart avec une valeur maximale prédéterminée de cet écart,
  - en fonction de cette comparaison, à l'étape pc) on ajuste le paramètre de réglage des conditions optiques d'acquisition du dispositif de capture d'image de manière à faire tendre ladite valeur mesurée vers ladite valeur cible de luminosité.

9. Procédé selon l'une des revendications 1 à 8, comportant à l'étape c), une étape k) de détection des groupes de pixels présentant un pic de luminosité isolé de taille inférieure à la taille de l'élément de calibrage de luminosité dans toutes les directions.

10. Procédé selon la revendication 9, selon lequel il est prévu une étape l) de sélection d'au moins deux groupes de pixels présentant un pic de luminosité isolé détectés à l'étape k) présentant la plus forte probabilité d'être associés à l'image de deux des éléments de calibrage de luminosité de l'accessoire, à savoir au moins deux groupes de pixels pour lesquels est réduit l'écart entre

- une distance mesurée entre ces deux groupes et une distance de référence, et/ou
- un angle mesuré entre la droite passant par ces deux groupes et une direction de référence et un angle de référence, et/ou
- la luminosité mesurée au voisinage de ces deux groupes et une luminosité de référence, et/ou
- la différence de luminosité entre deux points de positions relatives par rapport à chaque groupe de pixels prédéterminées et une différence de luminosité de référence.

11. Procédé selon la revendication 10, selon lequel on exclut de la sélection réalisée à l'étape l) les couples de deux groupes de pixels pour lesquels l'écart entre une distance mesurée entre ces deux groupes et une distance de référence, et/ou un angle mesuré entre la droite passant par ces deux groupes et une direction de référence et un angle de référence, et/ou la luminosité mesurée au voisinage de ces deux groupes et une luminosité de référence et/ou la différence de luminosité entre deux points de positions relatives par rapport à chaque groupe de pixels prédéterminées et une différence de luminosité de référence est supérieur à une valeur seuil.

12. Procédé selon l'une des revendications précédentes, selon lequel lesdites zones contrastées (ZS, ZC) de l'élément de calibrage (21D, 21G, 22H, 22B, 23, 24) de luminosité comportent une zone de gris sombre et une zone de gris clair.

13. Procédé selon l'une des revendications précédentes, selon lequel chaque élément de calibrage (21D, 21G, 22H, 22B, 23, 24) de luminosité comporte quatre zones contrastées (ZS, ZC) en alternance, chaque zone formant un angle droit de sommet commun avec les angles droits formés par les autres zones.

14. Procédé selon l'une des revendications précédentes, selon lequel l'accessoire (20) est fixé sur la monture (30) de lunettes du porteur.

**Patentansprüche**

1. Verfahren zur Bestimmung von mindestens einem geometrischen/physiognomischen Parameter im Zusammenhang mit der Montage eines Brillenglases in einer von einem Benutzer getragenen Brillenfassung (30), das die folgenden Schritte umfasst:

a) Anbringen, an dem Kopf (10) des Benutzers, eines Gestells (20), das mindestens ein Helligkeitskalibrierelement (21D, 21G, 22H, 22B, 23, 24), das mindestens zwei kontrastierende Zonen (ZS, ZC) beinhaltet, umfasst,
b) Erfassen eines Bildes (I1) des Kopfes (10) des Benutzers, an dem das Gestell (20) angeordnet ist, mit Hilfe einer Bilderfassungsvorrichtung,
c) Bestimmen der Position des Bildes des Helligkeitskalibrierelements (I21D, I21G, I22H, I22B, I23, I24) des Gestells 20 auf dem in Schritt b) erfassten Bild I1,
d) Bestimmen eines Helligkeitsmesswerts von mindestens einem Teil des Bildes dieses Helligkeitskalibrierelements (I21D, I21G, I22H, I22B, I23, I24), indem ein Helligkeitsmesswert jeder kontrastierenden Zone des Bildes dieses Helligkeitskalibrierelements bestimmt wird und indem der Mittelwert dieser zwei Werte bestimmt wird,
e) Verändern eines Parameters zum Einstellen der optischen Bildaufnahmebedingungen der Bilderfassungsvorrichtung in Abhängigkeit von diesem Messwert, um den Kontrast des Bildes der kontrastierenden Zonen (ZS, ZC) des Kalibrierelements zu verbessern,
f) Erfassen eines weiteren Bildes mit dem veränderten Einstellparameter für die optischen Aufnahmebedingungen und
g) Bestimmen des gesuchten geometrischen/physiognomischen Montageparameters anhand dieses weiteren Bildes.

2. Verfahren nach Anspruch 1, wobei nach dem Schritt e) die Schritte b) bis e) wiederholt werden, solange der in Schritt d) gemessene Helligkeitswert außerhalb eines Bereichs vorher festgelegter Werte liegt, und die Schritte f) und g) durchgeführt werden, wenn sich dieser Messwert innerhalb des vorher festgelegten Bereichs befindet.

3. Verfahren nach einem der Ansprüche 1 und 2, wobei, während das Gestell ferner ein Bezugselement umfasst, das mindestens eine bekannte geometrische Eigenschaft aufweist, in Schritt g) mindestens eine geometrische Eigenschaft dieses Bezugselements des Gestells in dem in Schritt f) erfassten Bild identifiziert wird.

**4.** Verfahren nach Anspruch 3, wobei das Bezugselement (21D, 21G, 22H, 22B, 23, 24) mit dem Helligkeitskalibrierelement (21D, 21G, 22H, 22B, 23, 24) zusammenfällt.

**5.** Verfahren nach einem der Ansprüche 1 bis 4, wobei sich in Schritt d) der Teil des Bildes des Helligkeitskalibrierelements, für den ein Helligkeitsmesswert bestimmt wird, zwischen den zwei kontrastierenden Zonen (ZS, ZC) befindet.

**6.** Verfahren nach einem der Ansprüche 1 bis 5, wobei in Schritt e)

- die Differenz zwischen dem in Schritt d) bestimmten Helligkeitsmesswert und einem Helligkeitszielwert bestimmt wird,
- diese Differenz mit einem vorher festgelegten Maximalwert dieser Differenz verglichen wird,
- in Abhängigkeit von diesem Vergleich der Einstellparameter für die optischen Aufnahmebedingungen der Bilderfassungsvorrichtung so verändert wird, dass sich der Messwert dem Helligkeitszielwert annähert.

**7.** Verfahren nach einem der Ansprüche 1 bis 6, das die folgenden Schritte, die dem Schritt b) vorausgehen, umfasst:

pa) Erfassen eines Vorabbildes des Kopfes des Benutzers,
pb) Bestimmen eines mittleren Helligkeitsmesswerts von mindestens einer reduzierten Zone dieses Vorabbildes, wobei diese reduzierte Zone dazu angepasst ist, mindestens einen Teil des Bildes des Kopfes des Benutzers abzudecken,
pc) grobes Justieren des Einstellparameters für die optischen Aufnahmebedingungen der Bilderfassungsvorrichtung in Abhängigkeit von diesem Messwert, um den Kontrast des erfassten Bildes des Kopfes des Benutzers zu verbessern,
pd) Verwenden des in Schritt pc) erhaltenen Einstellparameters für die optischen Aufnahmebedingungen, um die optischen Bedingungen für die Bilderfassung des Schritts b) zu justieren.

**8.** Verfahren nach Anspruch 7, wobei in Schritt pc)

- die Differenz zwischen dem in Schritt pb) bestimmten Helligkeitsmesswert und einem Helligkeitszielwert bestimmt wird,
- diese Differenz mit einem vorher festgelegten Maximalwert dieser Differenz verglichen wird,
- in Abhängigkeit von diesem Vergleich in Schritt pc) der Einstellparameter für die optischen Aufnahmebedingungen der Bilderfassungsvorrichtung so justiert wird, dass sich der Messwert dem Helligkeitszielwert annähert.

**9.** Verfahren nach einem der Ansprüche 1 bis 8, das in Schritt c) einen Schritt k) zur Detektion der Pixelgruppen umfasst, die eine isolierte Helligkeitsspitze aufweisen und deren Größe in allen Richtungen kleiner als die Größe des Helligkeitskalibrierelements ist.

**10.** Verfahren nach Anspruch 9, wobei ein Schritt l) zur Auswahl von mindestens zwei in Schritt k) detektierten Pixelgruppen, die eine isolierte Helligkeitsspitze aufweisen, vorgesehen ist, die die größte Wahrscheinlichkeit aufweisen, mit dem Bild von zwei der Helligkeitskalibrierelemente des Gestells assoziiert zu sein, nämlich mindestens zwei Pixelgruppen, bei denen die Differenz zwischen Folgendem reduziert ist:

- einem zwischen diesen zwei Gruppen gemessenen Abstand und einem Referenzabstand und/oder
- einem zwischen der Geraden, die durch diese zwei Gruppen verläuft, und einer Referenzrichtung gemessenen Winkel und einem Referenzwinkel und/oder
- der in der Nähe dieser zwei Gruppen gemessenen Helligkeit und einer Referenzhelligkeit und/oder
- dem Helligkeitsunterschied zwischen zwei Punkten von vorher festgelegten Relativpositionen mit Bezug auf jede Pixelgruppe und einem Referenzhelligkeitsunterschied.

**11.** Verfahren nach Anspruch 10, wobei aus der in Schritt l) durchgeführten Auswahl die Paare von zwei Pixelgruppen ausgeschlossen werden, bei denen die Differenz zwischen einem zwischen diesen beiden Gruppen gemessenen Abstand und einem Referenzabstand und/oder einem zwischen der Geraden, die durch diese zwei Gruppen verläuft, und einer Referenzrichtung gemessenen Winkel und einem Referenzwinkel und/oder der in der Nähe dieser zwei Gruppen gemessenen Helligkeit und einer Referenzhelligkeit und/oder dem Helligkeitsunterschied zwischen zwei Punkten von vorher festgelegten Relativpositionen mit Bezug auf jede Pixelgruppe und einem Referenzhelligkeitsunterschied größer als ein Schwellenwert ist.

**12.** Verfahren nach einem der vorhergehenden Ansprüche, wobei die kontrastierenden Zonen (ZS, ZC) des Helligkeitskalibrierelements (21D, 21G, 22H, 22B, 23, 24) eine dunkelgraue Zone und eine hellgraue Zone umfassen.

**13.** Verfahren nach einem der vorhergehenden Ansprüche, wobei jedes Helligkeitskalibrierelement (21D, 21G, 22H, 22B, 23, 24) vier sich abwechselnde kontrastierende Zonen (ZS, ZC) umfasst, wobei jede Zone einen rechten Winkel bildet, der einen gemeinsamen Scheitelpunkt mit den durch die anderen Zonen gebildeten rechten Winkeln hat.

14. Verfahren nach einem der vorhergehenden Ansprüche, wobei das Gestell (20) an der Brillenfassung (30) des Benutzers befestigt ist.

**Claims**

1. Method for determining at least one geometric/physiognomic parameter associated with the mounting of an ophthalmic lens in a spectacle frame (30) worn by a user, comprising the following steps:

> a) placing an accessory (20) on the user's head (10), said accessory comprising at least one brightness calibration element (21D, 21G, 22H, 22B, 23, 24) including at least two contrasted zones (ZS, ZC);
> b) capturing an image (I1) of the user's head (10) on which said accessory (20) has been placed, using an image capture device;
> c) determining the position of the image of the brightness calibration element (I21D, I21G, I22H, I22B, 123, 124) of said accessory (20) on said image (I1) captured in step b);
> d) determining a measured value of the brightness of at least one part of the image of this brightness calibration element (I21D, I21G, I22H, I22B, 123, 124), by determining a measured value of the brightness of each contrasted zone of the image of this brightness calibration element and calculating the average of these two values;
> e) modifying a parameter for adjusting the optical image-acquisition conditions of said image capture device as a function of this measured value in such a way as to improve the contrast of the image of the contrasted zones (ZS, ZC) of the calibration element;
> f) capturing another image with the modified parameter for adjusting the optical acquisition conditions; and
> g) determining said looked-for geometric/physiognomic mounting parameter on the basis of this other image.

2. Method according to Claim 1, according to which, after step e), steps b) to e) are repeated while the brightness value measured in step d) is outside an interval of predetermined values, and steps f) and g) are carried out when this measured value is located inside said predetermined interval.

3. Method according to one of Claims 1 and 2, according to which, the accessory furthermore comprising a reference element having at least one known geometric characteristic, in step g), at least one geometric characteristic of this reference element of the accessory is identified on the image captured in step f).

4. Method according to Claim 3, wherein said reference element (21D, 21G, 22H, 22B, 23, 24) is combined with said brightness calibration element (21D, 21G, 22H, 22B, 23, 24).

5. Method according to one of Claims 1 to 4, according to which, in step d), the part of the image of the brightness calibration element for which a measured value of the brightness is determined straddles said two contrasted zones (ZS, ZC).

6. Method according to one of Claims 1 to 5, according to which, in step e),

> - the difference between the measured brightness value determined in step d) and a target brightness value is determined,
> - this difference is compared with a predetermined maximum value of this difference,
> - according to this comparison, the parameter for adjusting the optical acquisition conditions of the image capture device is modified in such a way as to make said measured value extend towards said target brightness value.

7. Method according to one of Claims 1 to 6, comprising the following steps, which are preliminary to step b):

> pa) capturing a preliminary image of the user's head,
> pb) determining a measured value of the mean brightness of at least one reduced zone of this preliminary image, this reduced zone being suitable for covering at least a part of the image of the user's head,
> pc) roughly adjusting the parameter for adjusting the optical acquisition conditions of the image capture device according to this measured value in such a way as to improve the contrast of the captured image of the user's head,
> pd) using the parameter for adjusting the optical acquisition conditions obtained in step pc) to adjust the optical conditions of the image capture of step b).

8. Method according to Claim 7, according to which, in step pc),

> - the difference between the measured value of the brightness determined in step pb) and a target brightness value is determined,
- this difference is compared with a predetermined maximum value of this difference,
- according to this comparison, in step pc), the parameter for adjusting the optical acquisition conditions of the image capture device is adjusted in such a way as to make said measured value extend towards said target brightness value.

9. Method according to one of Claims 1 to 8, comprising, in step c), a step k) of detection of the groups of pixels having an isolated brightness peak smaller in size than the brightness calibration element in all directions.

10. Method according to Claim 9, according to which a step l) of selection of at least two groups of pixels having an isolated brightness peak detected in step k) is provided, having the highest probability of being associated with the image of two of the brightness calibration elements of the accessory, i.e. at least two groups of pixels for which the difference between

- a distance measured between these two groups and a reference distance, and/or
- an angle measured between the straight line passing through these two groups and a reference direction and a reference angle, and/or
- the brightness measured in the vicinity of these two groups and a reference brightness, and/or
- the brightness difference between two points of predetermined relative positions in relation to each group of pixels and a reference brightness difference is reduced.

11. Method according to Claim 10, according to which the pairs of two groups of pixels for which the difference between a distance measured between these two groups and a reference distance, and/or an angle measured between the straight line passing through these two groups and a reference direction and a reference angle, and/or the brightness measured in the vicinity of these two groups and a reference brightness and/or the brightness difference between two points of predetermined relative positions in relation to each group of pixels and a reference brightness difference is greater than a threshold value are excluded from the selection made in step l).

12. Method according to one of the preceding claims, according to which said contrasted zones (ZS, ZC) of the brightness calibration element (21D, 21G, 22H, 22B, 23, 24) comprise a dark gray zone and a light gray zone.

13. Method according to one of the preceding claims, according to which each brightness calibration element (21D, 21G, 22H, 22B, 23, 24) comprises four alternating contrasted zones (ZS, ZC), each zone forming a right angle with a common apex with the right angles formed by the other zones.

14. Method according to one of the preceding claims, according to which the accessory (20) is fixed onto the spectacle frame (30) of the user.

Fig.1

```
┌──────────────┐
│  Acquisition │ ⟍ 100'
│    vidéo     │
└──────┬───────┘
       │
       ▼
┌──────────────┐
│  Mesure de la│
│ luminosité sur│ ⟍ 200
│ une zone large│
│  de l'image  │
└──────┬───────┘
       │
       ▼                                    ┌──────────────┐
      ╱╲                          310 ⟍     │ Modification │
 300'╱  ╲        Test                        │  paramètres  │
    ╱    ╲────────────────────────────────▶ │ d'acquisition│◀─┐
    ╲    ╱     luminosité                    │    vidéo     │  │
     ╲  ╱                                    └──────┬───────┘  │
      ╲╱                                            │          │
       │ OK                                         │          │
       ▼                                            ▼          │
┌──────────────┐                                               │
│  Acquisition │◀──────────────────────────────────           │
100 │   vidéo    │                                             │
└──────┬───────┘                                               │
       │                                                       │
       ▼                                                       │
┌──────────────┐                                               │
│ Conversion en│                                               │
│    image     │ ⟍ 400                                         │
│ de luminance │                                               │
└──────┬───────┘                                               │
       │                                                       │
       ▼                                                       │
┌──────────────┐                                               │
│Rééchantillonnage│ ⟍ 500                                      │
└──────┬───────┘                                               │
       │                                                       │
       ▼                                                       │
┌──────────────┐                                               │
│ Détermination│                                               │
│ de la position│ ⟍ 600                                        │
│  des mires   │                                               │
└──────┬───────┘                                               │
       │                                                       │
       ▼                         700                           │
┌──────────────┐                      ┌────────────────────┐   │
│ Détermination précise│        900 ⟍ │     Mesure du      │   │
│ de la position│               │     │    paramètre       │   │
│  des mires   │               │     │géométrico-physionomique│ │
│ sur image non│                     └─────────▲──────────┘   │
│rééchantillonnée│                              │ OK           │
└──────┬───────┘                                │              │
       │                                        │              │
       ▼                                       ╱╲              │
┌──────────────┐                              ╱  ╲             │
│Mesure luminosité│                          ╱    ╲   Test      │
800 │ sur les mires │──────────────────────▶╲    ╱ luminosité──┘
└──────────────┘                             ╲  ╱
                                              ╲╱  ⟍ 300
```

I10

I22H

I2

I21D

I22B

I21G

I20

I30

I1

Fig.2

Fig.3

22H

20

26

27

25

21D

28

22B

23

24

28

ZC

ZS

ZS

ZC

21G

L

ZC

ZS

Fig.4A

21G

H

ZC

ZS

Fig.4B

Fig.5

Fig.6

**EP 2 678 735 B1**

## RÉFÉRENCES CITÉES DANS LA DESCRIPTION

**Documents brevets cités dans la description**

- WO 0184222 A

- WO 2006029875 A1